# EUROPEAN PATENT SPECIFICATION

(11) **EP 4 568 621 B1**
(45) Date of publication and mention of the grant of the patent: **01.04.2026**
(21) Application number: 23754882.1
(22) Date of filing: 01.08.2023
(51) Int. Cl.: A61F 5/44, A61F 5/445

(54) **URINE COLLECTION DEVICE AND METHOD OF USE THEREOF**
URINSAMMELVORRICHTUNG UND VERFAHREN ZU IHRER VERWENDUNG
DISPOSITIF DE COLLECTE D'URINE ET SA MÉTHODE D'UTILISATION

(30) Priority: 12.08.2022 LV 220069
(43) Date of publication of application: 18.06.2025
(73) Proprietor: Apex Dynamics, SIA, 1011 Riga (LV)
(72) Inventor: ATMATS, Uldis, 1011 Riga (LV)
(74) Representative: Kromanis, Artis
(86) International application number: PCT/LV2023/050012
(87) International publication number: WO 2024/035249

(56) References cited:
- CN-A- 105 716 911
- CN-A- 111 358 614
- CN-A- 113 925 663
- CN-U- 209 967 195
- GB-A- 2 581 831
- US-A- 2 496 175
- US-A- 3 421 504
- US-A- 4 529 402
- US-A- 6 021 531
- US-A1- 2005 075 615

## Description

### Field of the invention

The present invention relates to a urine collection device.

### Background of the invention

The prior art discloses various urine collection devices. International patent application publications Nos. WO2006044249 and WO2012118643, as well as Chinese utility model registration publications Nos. CN211246116U, CN211750679U and CN213697005U disclose some of urine collection devices.

Chinese utility model registration publications Nos. CN209316203U, CN209713782U, CN209967195U, upon which the preamble of claim 1 is based, and CN215273744U disclose systems that utilize urostomy pouches. One of the most substantial drawbacks is the complexity of these systems. They include additional pump systems and/or complex valve systems. These additional elements not only complicate the use of the devices, but also increase the exploitation expenditures or exploitation fee for the end user thereof.

Aforementioned urine collection devices do not provide safe and reliable drainage of urine from a user during night, sleeping in a bed. It is often the case that the user of the urostomy pouch may accidently squeeze said pouch during night sleep and leak of urine may arise.

The aim of the invention is to avoid the previously-mentioned complexities of the prior art. Moreover, the aim of the invention is to create a urine collection device that can be used by a user during night, sleeping in a bed without a worry that squeezing of the urostomy pouch may lead to leak of urine in a bed.

### Summary of the invention

The aim of the invention is reached by the design of a urine collection device comprising the following elements: a urostomy pouch, a urine collection bottle, and a flexible tube connecting the urostomy pouch with the urine collection bottle.

The urostomy pouch comprises a urostomy pouch hole for receiving urine from the human's urine discharge point or channel. The urostomy pouch further comprises a urine outlet opening for removing urine from the urostomy pouch. The urine outlet opening is provided with a valve so that the urostomy pouch can be closed or opened in order to discharge urine. The valve can be a manually-rotatable block valve. Any commercially-available urostomy pouch or any urostomy pouch known by the skilled person can be used as the urostomy pouch.

The flexible tube comprises a first end and a second end, where the first end of the flexible tube is connectable to the urostomy pouch opening, and the second end of the flexible tube is connectable to the urine collection bottle. Any commercially-available or well known to the skilled person, and medical-grade flexible tube can be used as the flexible tube.

The essence of the invention is the urine collection bottle of a special configuration allowing to create a negative pressure or such rarefaction in the urine collection device, that the discharged urine in the urostomy pouch can be safely transported to the urine collection bottle, and backflow of urine or unreasonable accumulation of urine in the urostomy pouch would not take place during the night-time - the time when the user of the urine collection device is sleeping. The urine collection bottle comprises a hole configured to be connected to the second end of the flexible tube in such a manner that a sealed fluid communication is provided between the urostomy pouch and the urine collection bottle. The urine collection bottle further comprises a corrugated wall formed such as to provide that the urine collection bottle is squeezable, decreasing its volume. The corrugated wall is formed such that it can return the urine collection bottle to its initial volume or state when the pressure between the urine collection device and the surrounding environment is equalized. Accordingly, the corrugation of the corrugated wall shall be formed such as to allow to squeeze the urine collection bottle, letting it to return to its initial state, thereby compensating the amount of incoming urine.

When the squeezed urine collection bottle is connected to the urostomy pouch through the flexible tube, and the urostomy pouch is connected to the human's or patient's urine collection point or channel, the entire device or system is in a sealed fluid communication and isolated from the surrounding environment.

The urine collection bottle with its bottle hole is configured to be connected to the urostomy pouch via the flexible tube in such a manner that a sealed fluid communication is provided between the urostomy pouch and the urine collection bottle. The urine collection bottle is characterized in that is comprises a corrugated wall formed such as to provide that said urine collection bottle is squeezable, decreasing its volume. The corrugated wall is formed such that it can return the urine collection bottle to its initial volume or state when the pressure between the urine collection device and the surrounding environment is equalized. The squeezed urine collection bottle connected to the urostomy pouch via the flexible tube forms negative pressure within the squeezed urine collection bottle, the urostomy pouch and the flexible tube, in result of which urine released by the user of the device is transferred from the urostomy pouch via flexible tube to the squeezed urine collection bottle. Hence, no urine is stored within the urostomy pouch and accidental release of urine from the urostomy pouch is prevented. The squeezed urine collection bottle gradually returns to its initial shape through a gradual release of urine by the user of the device.

The volume of the urine collection bottle in an unsqueezed state can be in the range from 2 to 3.5 litres, preferably about 2.7 litres. The volume of the urine collection bottle in a squeezed state, in its turn, may be in the range from 1 to 1.9 litres, preferably about 1.5 litres. Approximately a double change in the volume is sufficient so that the pressure in the urine collection device is lower enough than that of the surrounding environment, causing transportation of urine received in the urostomy pouch to the urine collection bottle. By analogy with practically all known nocturnal urine collection systems the urine collection bottle should be located below the user's sleeping level (i.e. - by the sleeping place).

In one embodiment of the invention the hole of the urine collection bottle and the second end of the flexible tube comprise a threaded connector, allowing the second end of the flexible tube to be screwed to the hole of the urine collection bottle, completely isolating said urine collection bottle from the surrounding environment. The device comprises a cap attached to the second end of the flexible tube and adapted to be screwed onto the hole of the urine collection bottle. Such a screw cap is very useful as it can be slightly opened before squeezing of the urine collection bottle, thus releasing excess air. A negative pressure or rarefaction is maintained in the urine collection bottle of the device by tightly screwing the cap afterwards, allowing to collect or transfer urine from the urostomy pouch to the urine collection bottle.

The aim of the invention is reached also through the method of use of the previously mentioned urine collection device. The method comprises the following steps: (I) squeezing the urine collection bottle be means of the corrugated wall of the urine collection bottle; (II) connecting the squeezed urine collection bottle to the urostomy pouch via the flexible tube creating fluidly connected and from environment sealed system of the urostomy pouch, the flexible tube and the urine collection bottle, and maintaining an air pressure in the urine collection bottle, the flexible tube, and the urostomy pouch that is lower than an air pressure of surrounding environment. In such a state, urine released by the user is immediately transferred from the urostomy pouch, where is a danger of accidental urine release, to the urine collection bottle, where urine is kept safely and remotely from the user. The safety is maintained by remote positioning of the urine collection bottle that is provided by means of the flexible tube.

The method further comprises the steps of: (III) keeping the valve of the urostomy pouch in a closed state, when the squeezed urine collection bottle is connected to the to the urostomy pouch through the flexible tube; and (IV) opening the valve of the urostomy pouch when the squeezed urine collection bottle is connected to the urostomy pouch via the flexible tube, thus maintaining the air pressure in the urine collection bottle, the flexible tube, and the urostomy pouch that is lower than the air pressure of the surrounding environment.

The step of connecting the squeezed urine collection bottle to the urostomy pouch via the flexible tube is performed by screwing the cap of the flexible tube onto the bottle hole of the squeezed urine collection bottle.

### A list of drawings

The drawings illustrate by way of example various embodiments of the invention falling within the scope of the invention defined by the claims.
Fig. 1 is a schematic representation of the urine collection device.
Fig. 2 illustrates one embodiment of the urine collection bottle (6).

### Detailed description of the embodiments

The embodiments of the invention are now described with reference to the figures to illustrate objectives, advantages, and efficiency of the present invention.

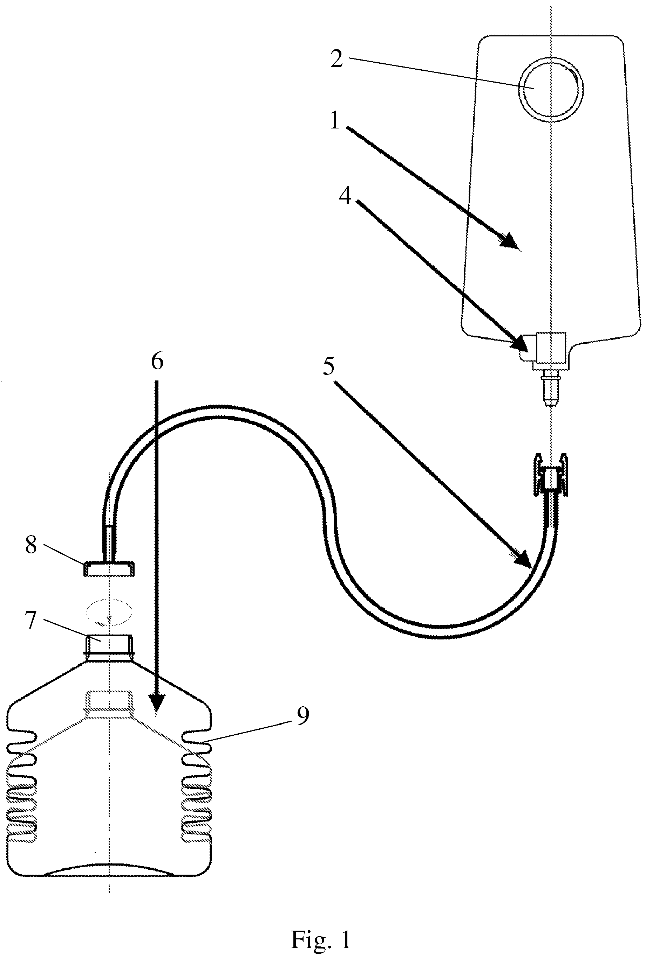

The urine collection device as seen in Fig. 1 comprises a urostomy pouch (1). The urostomy pouch (1) comprises a urostomy pouch hole (2) for receiving urine from a user or a patient, and a urine outlet opening (3) for removing urine from the urostomy pouch (1). The urine outlet opening (3) is further provided with a manually-rotatable block valve (4) for closing or opening of the urostomy pouch (1). The device comprises a flexible tube (5) comprising a first (15) and a second ends (51). The first end (15) of the flexible tube (5) is connected to the urine outlet opening (3) of the urostomy pouch (1). The device comprises a urine collection bottle (6) comprising a bottle hole (7). The bottle hole (7) of the urine collection bottle (6) is formed such as to be connectable to the second end (51) of the flexible tube (5) in such a manner that a sealed fluid communication is provided between the urostomy pouch (1) and the urine collection bottle (6). In the present embodiment of the invention the bottle hole (7) of the urine collection bottle (6) and the second end (51) of the flexible tube (5) comprise a threaded connector, allowing the second end (51) of the flexible tube (5) to be screwed to the hole (7) of the urine collection bottle (6), completely isolating or sealing said urine collection bottle (6) from the surrounding environment. This threaded connector is implemented by the device comprising a cap (8) attached to the second end of the flexible tube (5) and adapted to be screwed onto the bottle hole (7) of the urine collection bottle (6). The bottle hole (7) itself has a thread adapted to the thread of the cap (8). The urine collection bottle (6) itself comprises a corrugated wall (9) formed such as to provide that said urine collection bottle (6) is squeezable, decreasing its volume, wherein the corrugated wall (9) is formed such that it can return the urine collection bottle (6) to its initial volume or state when the pressure between the urine collection device and the surrounding environment is equalized. These elements are connected during use forming a closed urine collection environment with a negative pressure. In the present example the device is seen before its use when the urostomy pouch (1) and the urine collection bottle (6) are disconnected from the flexible tube (5). During a use of the device, the urine collection bottle (6) is squeezed and in this squeezable state attached to the flexible tube (5), thus creating a negative pressure within the urostomy pouch (1), the flexible tube (5) and the urine collection bottle (6). The negative pressure created withing the system allow forced transfer of urine released by the patient or user from the urostomy pouch (1) to the urine collection device (6).

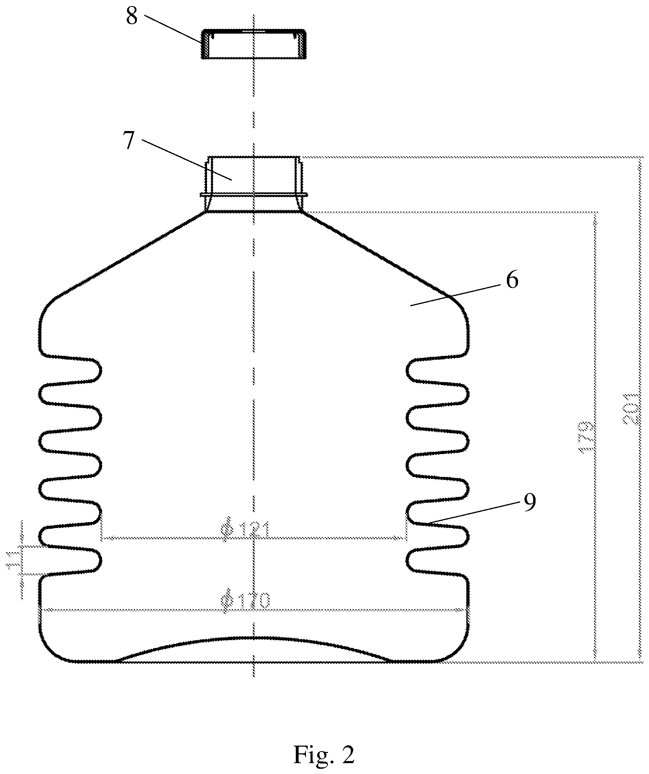

Fig. 2 illustrates one embodiment of the urine collection bottle (6). The urine collection bottle (6) comprises a bottle hole (7) being the only outlet point to the surrounding environment when the urine collection bottle (6) is not being used. During use this bottle hole (7) is in a fluid connection with the urostomy pouch (1) through the flexible tube (5). The hole (7) is closed with the cap (8) through the threaded connection thereof. There is a hole in the cap (8) itself for attaching the flexible tube (5). In this embodiment of the invention the urine collection bottle (6) has been presented with such dimensions where the height of the urine collection bottle (6) is 179 mm, however, the height of the bottle with the threaded connector of the bottle hole (7) is 201 mm. Moreover, in the present embodiment the corrugated wall (9) of the urine collection bottle (6) comprises four ridges or four protrusions, and five recesses therebetween, where the width of the recess is 11 mm. The diameter of the urine collection bottle (6) is 170 mm, however, the diameter of the bottle (6) between the recesses of the corrugated wall (9) is 121 mm. The volume of such a urine collection bottle (6) in an unsqueezed state is approximately 2.7 litres, but in a squeezed state - approximately 1.5 litres.

While the invention may be susceptible to various modifications and alternative forms, where some embodiments of the invention have been shown in the figures, it should be understood that the invention is not intended to be limited to the particular forms disclosed.

## Claims

1. A urine collection device comprising:
- a urostomy pouch (1), where the urostomy pouch (1) comprises a urostomy pouch hole (2) for receiving urine, and a urine outlet opening (3) for removing urine from the urostomy pouch (1), wherein the urine outlet opening (3) is provided with a valve (4) for closing and opening of the urostomy pouch (1),
- a flexible tube (5) comprising a first (15) and a second ends (51), where the first end (15) of the flexible tube (5) is connected to the opening (3) of the urostomy pouch (1),
- a urine collection bottle (6) comprising a bottle hole (7) configured to be connected to the second end (51) of the flexible tube (5) in such a manner that a sealed fluid communication is provided between the urostomy pouch (1) and the urine collection bottle (6), **characterized in that** the urine collection bottle (6) comprises a corrugated wall (9) formed such as to provide that said urine collection bottle (6) is squeezable, decreasing its volume allowing to create a negative pressure within the urostomy pouch (1), the flexible tube (5) and the squeezed urine collection bottle (6), in result of which urine released by a user of the device is forcibly transferred from the urostomy pouch (1) via flexible tube (5) to the squeezed urine collection bottle (6) and wherein the volume of the urine collection bottle (6) in an unsqueezed state is in the range from 2 to 3.5 litres, preferably about 2.7 litres, and the volume of the urine collection bottle (6) in a squeezed state is in the range from 1 to 1.9 litres, preferably about 1.5 litres.

2. The urine collection device according to claim 1, wherein the bottle hole (7) of the urine collection bottle (6) and the second end (51) of the flexible tube (5) comprise a threaded connector allowing the second end (51) of the flexible tube (5) to be screwed to the bottle hole (7) of the urine collection bottle (6), completely isolating said urine collection bottle (6) from the surrounding environment.

3. The urine collection device according to claim 2, wherein the device comprises a cap (8) attached to the second end of the flexible tube (5) and adapted to be screwed onto the bottle hole (7) of the urine collection bottle (6).

4. A method of use of the urine collection device according to any of claims 1 to 3 comprising the following steps:
- squeezing the urine collection bottle (6) using the corrugated wall (9) of the urine collection bottle (6);
- connecting the squeezed urine collection bottle (6) to the urostomy pouch (1) via the flexible tube (5) creating fluidly connected and from environment sealed system of the urostomy pouch (1), the flexible tube (5) and the urine collection bottle (6), and maintaining an air pressure in the urine collection bottle (6), the flexible tube (5), and the urostomy pouch (1) that is lower than an air pressure of surrounding environment,
wherein the method further comprises the steps of:
- keeping the valve (4) of the urostomy pouch (1) in a closed state, when the squeezed urine collection bottle (6) is connected to the to the urostomy pouch (1) through the flexible tube (5);
- opening the valve (4) of the urostomy pouch (1) when the squeezed urine collection bottle (6) is connected to the urostomy pouch (1) via the flexible tube (5), thus maintaining the air pressure in the urine collection bottle (6), the flexible tube (5), and the urostomy pouch (1) that is lower than the air pressure of the surrounding environment; and wherein the step of connecting the squeezed urine collection bottle (6) to the urostomy pouch (1) via the flexible tube (5) is performed by screwing the cap (8) of the flexible tube (5) onto the bottle hole (7) of the squeezed urine collection bottle (6).

## Patentansprüche

1. Eine Urinsammelvorrichtung, bestehend aus:
- einen Urostomiebeutel (1), wobei der Urostomiebeutel (1) eine Urostomiebeutelöffnung (2) zum Aufnehmen von Urin und eine Urinauslassöffnung (3) zum Entfernen von Urin aus dem Urostomiebeutel (1) umfasst, wobei die Urinauslassöffnung (3) mit einem Ventil (4) zum Schließen und Öffnen des Urostomiebeutels (1) versehen ist,
- einen flexiblen Schlauch (5) mit einem ersten (15) und einem zweiten Ende (51), wobei das erste Ende (15) des flexiblen Schlauchs (5) mit der Öffnung (3) des Urostomiebeutels (1) verbunden ist,
- eine Urinauffangflasche (6) mit einer Flaschenöffnung (7), die so konfiguriert ist, dass sie mit dem zweiten Ende (51) des flexiblen Schlauchs (5) verbunden werden kann, sodass eine dichte Flüssigkeitsverbindung zwischen dem Urostomiebeutel (1) und der Urinauffangflasche (6) hergestellt wird, **dadurch gekennzeichnet, dass** die Urinauffangflasche (6) eine gewellte Wand (9) aufweist, die so ausgebildet ist, dass die Urinauffangflasche (6) zusammendrückbar ist, wodurch ihr Volumen verringert wird, was die Erzeugung eines Unterdrucks innerhalb des Urostomiebeutels (1) ermöglicht, wobei der flexible Schlauch (5) und die zusammengedrückte Urinauffangflasche (6) so ausgebildet sind, dass der von einem Benutzer der Vorrichtung abgegebene Urin zwangsweise aus dem Urostomiebeutel (1) über den flexiblen Schlauch (5) in die zusammengedrückte Urinauffangflasche (6) übertragen wird, und wobei das Volumen der Urinauffangflasche (6) in einem nicht zusammengedrückten Zustand im Bereich von 2 bis 3,5 Litern, vorzugsweise etwa 2,7 Litern, liegt und das Volumen der Urinsammelkanne (6) in einem zusammengedrückten Zustand im Bereich von 1 bis 1,9 Litern, vorzugsweise etwa 1,5 Litern, liegt.

2. Die Urinsammelvorrichtung gemäß Anspruch 1, wobei die Flaschenöffnung (7) der Urinsammel-Flasche (6) und das zweite Ende (51) des flexiblen Schlauchs (5) einen Gewindeanschluss aufweisen, der es ermöglicht, das zweite Ende (51) des flexiblen Schlauchs (5) an die Flaschenöffnung (7) der Urinsammel-Flasche (6) anzuschrauben, wodurch die Urinsammelvorrichtung (6) vollständig von der Umgebung isoliert wird.

3. Die Urinsammelvorrichtung gemäß Anspruch 2, wobei die Vorrichtung eine Kappe (8) umfasst, die am zweiten Ende des flexiblen Schlauchs (5) angebracht ist und auf das Flaschenloch (7) der Urinsammel-Flasche (6) geschraubt werden kann.

4. Verfahren zur Verwendung der Urinsammelvorrichtung gemäß einem der Ansprüche 1 bis 3, das die folgenden Schritte umfasst:
- Zusammendrücken der Urinsammelkanne (6) unter Verwendung der gewellten Wand (9) der Urinsammelkanne (6);
- Verbinden der zusammengedrückten Urinsammelkanne (6) mit dem Urostomiebeutel (1) über den flexiblen Schlauch (5), wodurch ein fluidisch verbundenes und gegenüber der Umgebung abgedichtetes System aus dem Urostomiebeutel (1), - den flexiblen Schlauch (5) und die Urinsammelkanne (6) zu verbinden und einen Luftdruck in der Urinsammelkanne (6), dem flexiblen Schlauch (5) und dem Urostomiebeutel (1) aufrechtzuerhalten, der niedriger ist als der Luftdruck der Umgebung,
wobei das Verfahren ferner die folgenden Schritte umfasst:
- Halten des Ventils (4) des Urostomiebeutels (1) in einem geschlossenen Zustand, wenn die zusammengedrückte Urinsammelkanne (6) über den flexiblen Schlauch (5) mit dem Urostomiebeutel (1) verbunden ist;
- Öffnen des Ventils (4) des Urostomiebeutels (1), wenn die zusammengedrückte Urinsammelkanne (6) über den flexiblen Schlauch (5) mit dem Urostomiebeutel (1) verbunden ist, wodurch der Luftdruck in der Urinsammelkanne (6), dem flexiblen Schlauch (5) und dem Urostomiebeutel (1) aufrechterhalten wird, der niedriger ist als der Luftdruck der Umgebung; und wobei der Schritt des Verbindens der zusammengedrückten Urinsammel Flasche (6) mit dem Urostomiebeutel (1) über den flexiblen Schlauch (5) durchgeführt wird, indem die Kappe (8) des flexiblen Schlauchs (5) auf die Flaschenöffnung (7) der zusammengedrückten Urinsammel Flasche (6) geschraubt wird.

## Revendications

1. Dispositif de collecte d'urine comprenant:
- une poche d'urostomie (1), la poche d'urostomie (1) comprenant un orifice (2) destiné à recevoir l'urine et une ouverture (3) destinée à évacuer l'urine de la poche d'urostomie (1), l'ouverture (3) étant munie d'une valve (4) permettant de fermer et d'ouvrir la poche d'urostomie (1),
- un tube flexible (5) comprenant une première extrémité (15) et une deuxième extrémité (51), la première extrémité (15) du tube flexible (5) étant reliée à l'ouverture (3) de la poche d'urostomie (1),
- un flacon collecteur d'urine (6) comprenant un orifice (7) configuré pour être relié à la deuxième extrémité (51) du tube flexible (5) de manière à assurer une communication étanche entre la poche d'urostomie (1) et le flacon collecteur d'urine (6), **caractérisé en ce que** le flacon de collecte d'urine (6) comprend une paroi ondulée (9) formée de manière à permettre au flacon de collecte d'urine (6) d'être comprimé, ce qui réduit son volume et permet de créer une pression négative à l'intérieur de la poche d'urostomie (1), le tube flexible (5) et le flacon de collecte d'urine comprimé (6), ce qui a pour résultat que l'urine libérée par un utilisateur du dispositif est transférée de force de la poche d'urostomie (1) via le tube flexible (5) vers le flacon de collecte d'urine comprimé (6) et dans lequel le volume du flacon de collecte d'urine (6) à l'état non compressé est compris entre 2 et 3,5 litres, de préférence environ 2,7 litres, et le volume du flacon de collecte d'urine (6) à l'état compressé est compris entre 1 et 1,9 litre, de préférence environ 1,5 litre.

2. Dispositif de collecte d'urine selon la revendication 1, dans lequel l'orifice (7) du flacon de collecte d'urine (6) et la deuxième extrémité (51) du tube flexible (5) comprennent un raccord fileté permettant à la deuxième extrémité (51) du tube flexible (5) d'être vissée sur l'orifice (7) du flacon de collecte d'urine (6), isolant complètement ladite bouteille de collecte d'urine (6) de l'environnement environnant.

3. Dispositif de collecte d'urine selon la revendication 2, dans lequel le dispositif comprend un bouchon (8) fixé à la deuxième extrémité du tube flexible (5) et adapté pour être vissé sur l'orifice (7) du flacon de collecte d'urine (6).

4. Procédé d'utilisation du dispositif de collecte d'urine selon l'une quelconque des revendications 1 à 3, comprenant les étapes suivantes:
- presser le flacon de collecte d'urine (6) à l'aide de la paroi ondulée (9) du flacon de collecte d'urine (6);
- raccorder le flacon de collecte d'urine (6) pressé à la poche d'urostomie (1) via le tube flexible (5), créant ainsi un système raccordé de manière fluide et étanche à l'environnement de la poche d'urostomie (1), le tube flexible (5) et le flacon de collecte d'urine (6), et maintenir une pression d'air dans le flacon de collecte d'urine (6), le tube flexible (5) et la poche d'urostomie (1) qui est inférieure à la pression d'air de l'environnement environnant,
le procédé comprenant en outre les étapes suivantes:
- maintenir la valve (4) de la poche d'urostomie (1) dans un état fermé, lorsque le flacon de collecte d'urine pressé (6) est connecté à la poche d'urostomie (1) par l'intermédiaire du tube flexible (5);
- ouvrir la valve (4) de la poche d'urostomie (1) lorsque le flacon de collecte d'urine comprimé (6) est connecté à la poche d'urostomie (1) via le tube flexible (5), maintenant ainsi la pression atmosphérique dans le flacon de collecte d'urine (6), le tube flexible (5) et la poche d'urostomie (1) inférieure à la pression atmosphérique ambiante ; et dans lequel l'étape consistant à raccorder le flacon de collecte d'urine comprimé (6) à la poche d'urostomie (1) via le tube flexible (5) est réalisée en vissant le bouchon (8) du tube flexible (5) sur l'orifice (7) du flacon de collecte d'urine comprimé (6).
